# EUROPEAN PATENT APPLICATION

(11) **EP 2 181 646 A1**
(43) Date of publication of application: **05.05.2010**
(21) Application number: 09252324.0
(22) Date of filing: 30.09.2009
(51) Int. Cl.: A61B 1/32

(54) **Speculum**

(30) Priority: 29.10.2008 GB 0819796
(71) Applicant: Pelican Healthcare Limited, Cardiff CF14 5WF (GB)
(72) Inventor: Clark, Howard K., Cardiff CF14 5WF (GB)
(74) Representative: Davies, Gregory Mark

(57) **Abstract**

A speculum device for dilating a body cavity or orifice, the speculum device comprising a plurality of separator elements configured for relative movement between a relatively contracted configuration and a relatively expanded configuration and an actuator arrangement arranged to apply an opening force to urge reconfiguration of the speculum device to the expanded configuration against the resistance force applied by the body cavity or orifice, the device including a force limitation means arranged to inhibit the opening force from exceeding a threshold level.

## Description

A speculum is a commonly-used medical tool for opening or distending an orifice or cavity of a patient to permit examination of the interior and/or to enable a sample to be taken.

Various forms of specula are known, their designs varying in accordance with the body cavity to be inspected (e.g. vagina, rectum, ear, nostril). However, all forms of specula incorporate the same basic concept: elongate separator elements ('paddles', or 'blades') having a curved cross section, are positioned edge to edge so that in combination they form an elongate channel. The channel narrows towards the end to be inserted into the orifice to aid the insertion process whilst remaining relatively wider at the opposing end to facilitate visual examination or insertion of an instrument.

The two blades are hinged at their broader ends, thus resembling a duck's beak. Thus, the speculum is able to move to move between a closed configuration (i.e. blade edges are adjacent to each other) and an open configuration (i.e. blades are angled away from each other). Handles are attached, usually integrally, at the hinged ends. Squeezing of the handles causes the blades to move apart thus dilating the orifice or cavity and enabling examination. As the practitioner often needs to keep both hands free for performance of the examination, the speculum typically has a locking mechanism which enables the blades of the speculum to be held apart at a desired distance or angle without the need for the practitioner to hold the handles.

Traditionally, specula have been made of metal. Whilst this provides a sturdy and robust instrument, their manufacture can be expensive. In addition, patients can find the metal to be cold and thus the specula may need to be warmed before use. After use, a metal speculum must be sterilised before it can be used on another patient. All of these factors tend to increase cost in relation to metal specula.

As a result, specula are now commonly made of plastic. Such specula are warmer to the touch and thus more comfortable for the patient. They are often of transparent plastic, facilitating visual examination of the cavity. They are also single-use items which do not require sterilisation after use. These factors reduce cost and time requirements.

However, plastic blades are not as robust or sturdy as their metal counterparts. This is a drawback because as the blades of the speculum are spaced progressively apart, increasing pressure is exerted upon them by the resisting cavity/orifice walls. The plastics material is typically brittle (in order to prevent deflection of the blades during use). In some cases, it has been known for the blades to fracture. This not only prolongs the examination process but can cause distress, discomfort or even harm to the patient.

The arrangement described in PCT/2007/122618 attempts to address this problem, stating that due, to the risk of distortion or fracture of the speculum components, plastic specula are not suitable for heavy-duty use or prolonged gynecological procedures as they cannot withstand loads greater than 2 to 3 kg. However, the arrangement described attempts to solve the problem by reinforcing (e.g. thickening) the speculum components or manufacturing them from a more stress-resilient material such as polycarbonate. However, the fundamental problem associated with plastic specula is not addressed by this arrangement - i.e. the pressure is not released or reduced once it reaches a dangerous level, the danger threshold is simply raised. Indeed, PCT/2007/122618 claims an advantage that if the speculum does still fracture under extreme stress, this does not occur in regions whereby the patient is endangered. Thus the problem of avoiding breakage of the blades is not solved by PCT/2007/122618.

Thus, it is an object of the present invention to provide a speculum which overcomes at least this drawback, and eliminates or at least alleviates the possibility of the speculum blades fracturing, flexing or breaking under pressure during use.

Thus in accordance with the present invention, there is provided a speculum device for dilating a body cavity or orifice, the speculum device comprising:
a plurality of separator elements configured for relative movement between a relatively contracted configuration and a relatively expanded configuration;
an actuator arrangement arranged to apply an opening force to urge reconfiguration of the speculum device to the expanded configuration against the resistance force applied by the body cavity or orifice;
a force limitation means arranged to inhibit the opening force from exceeding a threshold level.

The threshold opening force is typically arranged to be at a level below the level of opening force at which the separator elements fail or fracture.

In one embodiment, the force limitation arrangement comprises the actuator arrangement.

In one embodiment, the actuator arrangement ceases to be capable of continuing to act to urge the speculum device toward the expanded configuration when the opening force reaches the threshold level.

In one embodiment the actuator arrangement is caused to fail when the opening force reaches the threshold level.

In one embodiment the actuator arrangement comprises a threaded shaft and a threaded member (for example a nut) mounted for rotation on the shaft; relative driving rotation of the threaded shaft and threaded member effecting the opening force to urge reconfiguration of the speculum device to the expanded configuration. The threaded member is typically finger actuated by a user.

Beneficially, the threaded engagement relationship between the threaded shaft and the threaded member is caused change (for example to fail, be rendered inactive, or overcome) when the opening force reaches the threshold level.

In one embodiment, the shaft includes a first threaded portion having a thread of a first quality and a second threaded portion having a thread of a second quality.

For example the shaft may include a first threaded portion having a thread of a first thread diameter and a second threaded portion having a thread of a second thread diameter. The lesser diameter thread may be positioned at a location on the shaft intended to be reached by threaded member when the threshold opening force is likely to be achieved. When the threshold force is achieved the threaded member slides translationally back over the threaded shaft.

It is possible to make the threaded connection between the threaded member and the shaft such that the threaded length of the shaft is all of one quality but the thread of the threaded member is selected such that the threaded shaft and threaded member can engage to open the separator elements up to the predetermined threshold force, but the threaded member is caused to slide back over the shaft when the threshold force is achieved.

This effect may be achieved also by ensuring that at least a portion of the thread of the shaft is intermittent.

Alternatively (or additionally) the thread may be made resiliently deformable, such that the thread deforms when the predetermined threshold force is attained.

In an alternative arrangement the actuator may include ratchet force limitation arrangement.

Typically, the separator elements comprise paddles or blades. Beneficially separator elements are provided with respective handle extensions extending transversely with respect to the respective separator element.

Beneficially the actuator arrangement is coupled to the handle extensions to move the handle elements with respect to one another.

The speculum is typically generally of moulded plastics construction.

In a typical embodiment of the invention, a speculum has a plurality of separator elements comprising elongate blades (or 'paddles' or 'limbs') which are used to push against the inner walls of a patient's cavity or orifice, thus dilating said cavity and permitting examination by a medical practitioner or other user. Typically, there are two such dilation blades although in some embodiments more blades may be incorporated (for example, for use with speculums designed for the examination of obese patients).

Typically, the blades are pivoted or hinged at one end to provide a coupling which facilitates relative angular separation of the blades. In other words, the blades can pivot around the hinge so that the distal ends move away from, or towards, each other. In a closed configuration, the elongate edges of the blades abut against one another. In an expanded/open configuration, the blades are spaced apart (separated) i.e. they are angularly displaced. The angle between the hinged blades can vary according to the practitioner's need. (It should be noted that in some known speculum arrangements, wherein the blades are coupled by means other than a hinge, the movement may be lateral rather than angular).

According to a typical embodiment, the blades are oriented into an upper and lower arrangement, although it will be understood by the skilled addressee that the blades may be otherwise oriented in accordance with other forms of embodiment.

Typically, the blades are conjoined, coupled or integrally formed with respective handles. The handles typically extend downwards, projecting substantially transversely from the hinged end of the blades. Typically, the handles extending from the upper and lower blades are arranged and positioned to form rear and front handles, respectively.

In a broad aspect the invention encompasses force limitation means of various types and configurations.

These and other aspects of the present invention will now be described by way of example only and with reference to the accompanying drawings in which:
Figure 1 is a side view of a typical speculum, the speculum being in a closed configuration;
Figure 2a is a side view of a shaft for use with a locking nut as known in the prior art. The thread of the shaft is of a uniform height along the entire length of the shaft;

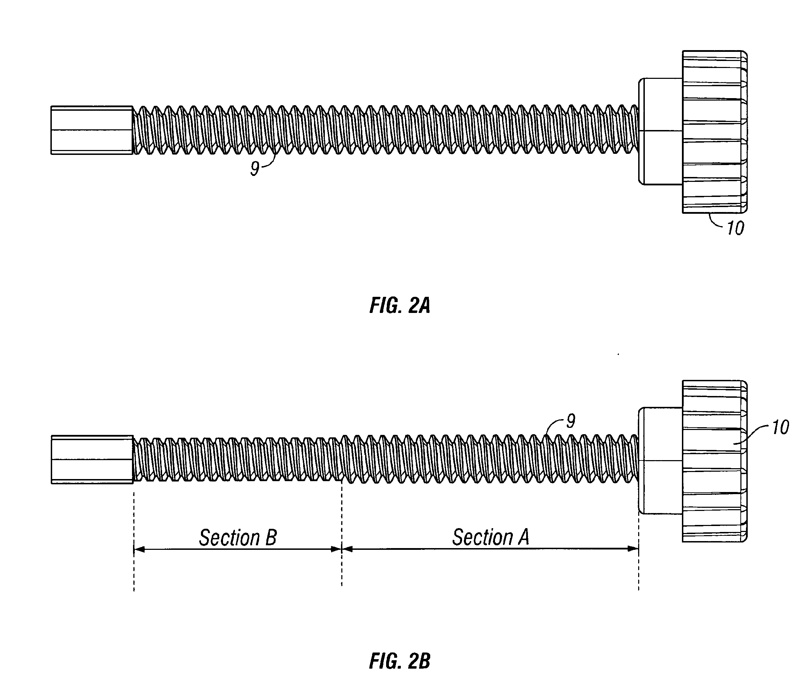

Figure 2 b is a side view of a shaft for use with a locking nut according to the present invention. The thread of the shaft is not of a uniform height along the entire length of the shaft;

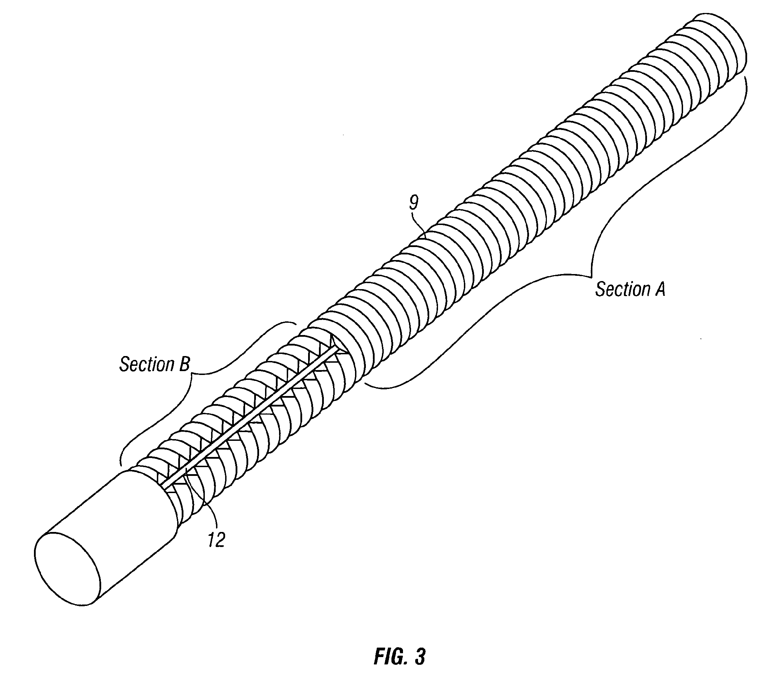

Figure 3 is a perspective view of an alternative embodiment of a shaft for use in accordance with the invention; and

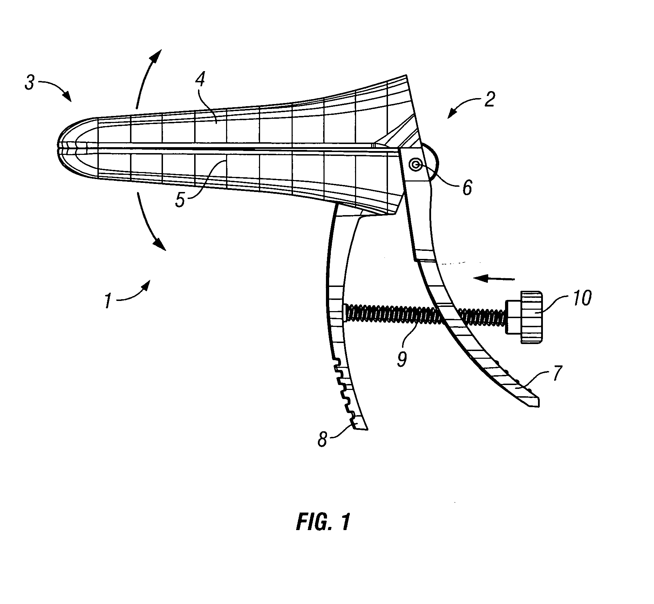

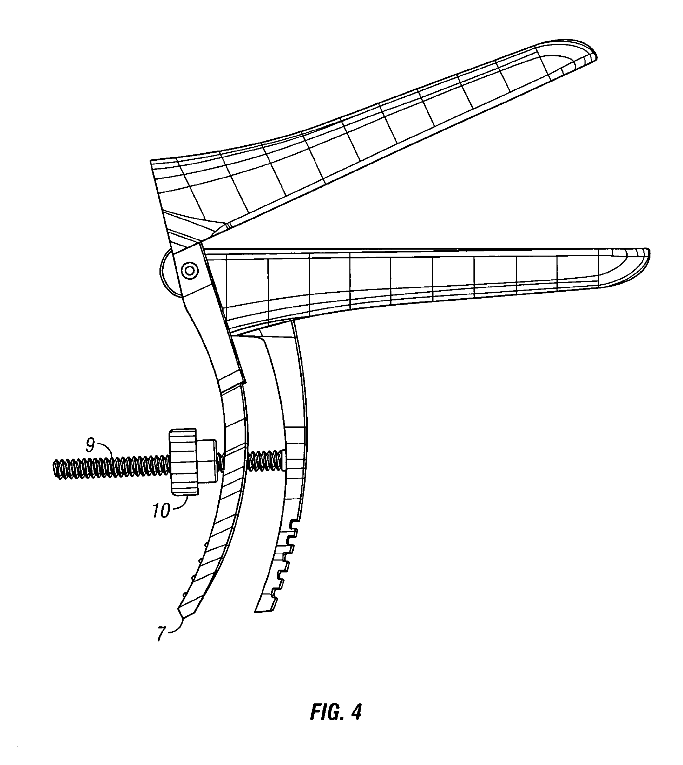

Figure 4 is a perspective view of the speculum of figure 1 in open (expanded) configuration.

Referring initially to figure 1, there is shown a vaginal speculum 1 of the type commonly used for dilating the vaginal cavity or orifice of a patient during examination or medical procedure. Such specula are often manufactured from a plastic material, having a plurality of curved separator elements typically referred to as 'blades' or 'paddles'. In a typical arrangement, there are two blades (4, 5) oriented such that one provides an upper blade 4 and the other provides a lower blade 5.

The end of the speculum which will be closest to the practitioner during use of the speculum is known hereafter as the 'proximal end' 2. The other end of the speculum, which is inserted into the cavity, is known hereafter as the 'distal end' 3.

The blades 4, 5 are coupled such that they can move towards or away from each other. Depending on the coupling arrangement used, this may be angular or lateral movement. In a typical arrangement, as shown in Figure 1, the coupling is a hinge 6 provided at the proximal end 2 of the speculum 1, such that the blades 4, 5 may pivot around the hinge 6 at their proximal ends 2 thus moving the distal ends 3 away from or towards each other.

Thus, the speculum 1 is moveable between a closed configuration (as shown in Figure 1) or in an open (expanded) configuration in which there is angular separation displacement of the blades 4, 5.

The blades 4, 5 are provided, coupled integrally or otherwise, with handles 7, 8. Upper blade 4 is shown as coupled to rear handle 7, and lower blade 8 is coupled to front handle 8. Pivoting the handles 7, 8 together thus causes the blades 4, 5 to move apart into an open (expanded) configuration.

A mechanism is provided to enable the practitioner to hold the speculum 1 open at a desired angle without having to use his/her hands to maintain pressure on the handles 7, 8. In the embodiment shown in figure 1, this mechanism comprises a threaded shaft 9 and cooperating threaded nut 10. The shaft 9 is coupled to the front handle 8 and passes through an aperture or bore in the rear handle 9 before the nut 10 is screwed onto the shaft 9.

Continued rotation of the nut 10 causes it to travel along the threaded shaft 9 until it comes into contact with the rear handle 7. At this point, continued rotation causes further progression of the nut 10 along the threaded shaft, the nut 10 pushing the rear handle 7 towards the front handle 8 and thus progressively opening the blades 4, 5. Thus the nut can be used as an actuator to apply opening force to the speculum via the handles 7,8.

In a known arrangement, the thread of shaft 9 is uniform and of a constant height along the length of the shaft 9. This is shown in Figure 2a.

However, Figure 2b illustrates a shaft 9 configured in accordance with the present invention to provide a force limitation facility. In this embodiment this facility is achieved because the thread of the shaft 9 is not of a uniform height/diameter along the entire shaft length. Figure 2b shows shaft 9 is provided with thread of different diameters. As most clearly illustrated in Figure 2b there is provided a plurality of sections along the length of shaft 9, each section having a thread of different height. Section A is positioned towards to the proximal end 2 of the speculum 1, whereas section B is towards the distal end 3 of the speculum. Section B has a reduced thread height in relation to section A. When the nut 10 is rotated to drivingly engage with the shaft at section A, the driving engagement is at its best. When the nut passes onto section b, the driving engagement of the nut with the thread is still in effect but, because of the reduced thread diameter I section B, above a threshold force level, the driving engagement fails and the nut can be forced backwards translationally over the thread in section B toward section A. This causes the 4,5 to move back towards the closed position, reducing the force applied to the blades by the vaginal orifice. The section B is strategically placed with respect to the position of section A, such that section b is encountered by the nut 10 when it is at a position on the shaft corresponding to an opening configuration at which the threshold force is likely to be encountered. The threshold force is designed to be reached before the blades fracture. The slight mismatch between the thread diameter of section B of the shaft and the nut thread permits the nut to move translationally over the section B thread when the threshold force is reached.

It will be appreciated that a similar effect may be achieved where the thread is not split into two or more sections, but the same in quality (thread diameter) along the relevant portions of the shaft. In this embodiment the thread diameter is selected with respect to the nut 10 so as to have the same effect as the relationship between the section B thread and the nut 10. Namely that the nut will drivingly engage with the threaded shaft 9 up to a predetermined threshold level, but upon reaching the threshold force, the nut will slide backwardly over the threaded shaft. This embodiment may be less preferred because the threaded driving engagement is not so smooth over the length of the shaft towards the proximal end 2 of the device because of the minor mismatch in the diameter of the threads of the nut 10 and shaft 9, in a region of the shaft where this thread diameter mismatch is not required.

A similar effect may be achieved where the thread is arranged to shear when the threshold force is reached. Additionally the thread may be deformable (resiliently or otherwise) when the threshold is reached.

For example in the arrangement of figure 3, the section B portion of the shaft 9 is not of reduced diameter, but rather the thread is intermittent around the circumference being provided with spaced elongate grooves 12 (only one is shown in the drawing). This arrangements means that the thread crests in section B are more easily deflectable, and permit the nut to slide translationally over the shaft back towards section A when the threshold force is reached.

The various thread qualities and characteristics that permit the threaded connection to be overcome may be combined in a single embodiment, fort example having resilient, intermittent, and mismatched thread diameters (in any combination).

The skilled addressee will also appreciate that the present invention can be configured and arranged to operate with a variety of forms of actuator mechanisms, force limitation arrangements and/or blade couplings (rather than with a hinged arrangement). For example, a ratchet and pawl mechanism may be used, wherein the teeth of the ratchet replace the thread of the shaft 9 described above. Some of the teeth may be of a reduced height, or deformable or shearable as described above in relation to the thread of shaft 9.

It should be noted that the above-mentioned embodiments illustrate rather than limit the invention, and that those skilled in the art will be capable of designing many alternative embodiments without departing from the scope of the invention as defined by the appended claims. In the claims, any reference signs placed in parentheses shall not be construed as limiting the claims. The word "comprising" and "comprises", and the like, does not exclude the presence of elements or steps other than those listed in any claim or the specification as a whole. In the present specification, "comprises" means "includes or consists of" and "comprising" means "including or consisting of". The singular reference of an element does not exclude the plural reference of such elements and vice-versa. In a device claim enumerating several means, several of these means may be embodied by one and the same item of hardware. The mere fact that certain measures are recited in mutually different dependent claims does not indicate that a combination of these measures cannot be used to advantage.

## Claims

1. A speculum device for dilating a body cavity or orifice, the speculum device comprising:
a plurality of separator elements configured for relative movement between a relatively contracted configuration and a relatively expanded configuration;
an actuator arrangement arranged to apply an opening force to urge reconfiguration of the speculum device to the expanded configuration against the resistance force applied by the body cavity or orifice;
the device including a force limitation means arranged to inhibit the opening force from exceeding a threshold level.

2. A speculum device according to claim 1 wherein the threshold opening force is arranged to be at a level below the level of opening force at which the separator elements fail or fracture.

3. A speculum device according to claim 1 or claim 2, wherein the force limitation arrangement comprises the actuator arrangement.

4. A speculum device according to any preceding claim wherein the actuator arrangement ceases to be capable of continuing to act to urge the speculum device toward the expanded configuration when the opening force reaches the threshold level.

5. A speculum device according to any preceding claim, wherein the actuator arrangement is caused to fail when the opening force reaches the threshold level.

6. A speculum device according to any preceding claim, wherein the actuator arrangement comprises a threaded shaft and a threaded member mounted for rotation on the shaft; relative rotation of the threaded shaft and threaded member effecting the opening force to urge reconfiguration of the speculum device to the expanded configuration.

7. A speculum device according to claim 6 wherein the threaded engagement relationship between the threaded shaft and the threaded member is caused change (for example to fail, be rendered inactive, or overcome) when the opening force reaches the threshold level.

8. A speculum device according to claim 6 or claim 7, wherein the shaft includes a first threaded portion having a thread of a first quality and a second threaded portion having a thread of a second quality.

9. A speculum device according to claim 8 wherein the shaft includes a first threaded portion having a thread of a first thread diameter and a second threaded portion having a thread of a second thread diameter.

10. A speculum device according to any of claims 6 to 9, wherein at least a portion of the thread of the shaft is intermittent.

11. A speculum device according to any of claims 6 to 10, wherein at least a portion of the thread of the shaft is resiliently deformable.

12. A speculum device according to any of claims 6 to 11, wherein at least a portion of the thread of the shaft has a non threaded section.

13. A speculum device according to any preceding claim, wherein the separator elements comprise paddles or blades.

14. A speculum device according to any preceding claim, wherein each separator element is provided with a respective handle extension extending transversely to the respective separator element.

15. A speculum device according to any preceding claim, wherein the separator elements are of moulded plastics material.
